# EUROPEAN PATENT APPLICATION

(11) **EP 3 299 023 A1**
(43) Date of publication of application: **28.03.2018**
(21) Application number: 16795914.7
(22) Date of filing: 20.05.2016
(51) Int. Cl.: A61K 35/28, A61P 37/02, A61P 29/00, A61P 25/00, A61P 1/00

(54) **LOW-OXYGEN-TREATED MESENCHYMAL STEM CELL AND USE THEREOF**

(30) Priority: 21.05.2015 CN 201510264541
(71) Applicant: Shanghai Institutes for Biological Sciences, Chinese Academy of Sciences, Shanghai 200031 (CN)
(72) Inventor: SHI, Yufang, Shanghai 200031 (CN); DU, Liming, Shanghai 200031 (CN); LIN, Liangyu, Shanghai 200031 (CN); WANG, Ying, Shanghai 200031 (CN)
(74) Representative: Dupuis-Latour, Dominique
(86) International application number: PCT/CN2016/082787
(87) International publication number: WO 2016/184427

(57) **Abstract**

The present invention relates to a hypoxia-treated mesenchymal stem cell and the use thereof. The present invention discloses for the first time that treating mesenchymal stem cells with hypoxia can significantly promote alleviating or therapeutic effects of mesenchymal stem cells on inflammatory diseases. The present invention also discloses that the hypoxia-treated mesenchymal stem cell is capable of producing insulin-like growth factor-2, which plays a central role in the treatment of inflammatory diseases with the hypoxia-treated mesenchymal stem cell.

## Description

### Technical Field

The present invention belongs to the field of biological medicines. More specifically, the present invention relates to a hypoxia-treated mesenchymal stem cell and the use thereof.

### Background Art

Mesenchymal stem/stromal cells (MSCs), also known as tissue stem cells, have the ability of self renewal and multi-directional differentiation. In almost all tissues in the body, MSCs are able to self renew and differentiate into specific tissue cells so as to repair tissue damage.

At present, MSCs that are *in vitro* isolated and cultured have been used to treat various immune-associated animal disease models or clinical diseases, and the effectiveness and safety thereof have been certified. However, different studies have different interpretations on the mechanisms of how they exert therapeutic effects. It has been reported that MSCs can secrete a series of growth factors such as hepatocyte growth factor (HGF), epidermal growth factor (EGF) and transforming growth factor (TGF-β), and the role of these factors in disease treatment with MSCs still needs further discussion.

Mesenchymal stem cells are considered to have broad application prospects. However, in view of the complexity of disease microenvironments in the body and the continual emergence of ineffective and failed cases of MSC treatment, there is still a need to explore deeper interactions and regulation mechanism of disease microenvironments and MSCs, so as to make MSCs-mediated stem cell treatment more available not only in laboratory but clinical practice.

### Summary of the Invention

An object of the present invention is to provide a hypoxia-treated mesenchymal stem cell and the use thereof.

In a first aspect of the present invention, provided is the use of a hypoxia-treated mesenchymal stem cell or a cell culture or culture supernatant thereof in the manufacture of a medicament for preventing, alleviating or treating inflammatory diseases.

In a preferred embodiment, the inflammatory diseases include: multiple sclerosis or inflammatory bowel disease (IBD).

In another preferred embodiment, the hypoxia is 1% to 15%, preferably 1% to 13%, more preferably 5% to 10% of oxygen by volume.

In another preferred embodiment, the hypoxia-treated mesenchymal stem cell or the cell culture or culture supernatant thereof refers to: the obtained mesenchymal stem cell or cell culture or culture supernatant thereof being continuously cultured under conditions of 1% to 15%, preferably 1% to 13%, more preferably 5% to 10% of oxygen in terms of volume ratio. Preferably, the culturing is lasted for more than two passages, and more preferably for more than three passages, such as from 3 to 20 passages, from 3 to 10 passages, and from 3 to 5 passages.

In another preferred embodiment, the medicament is also used for:
increasing the proportion of regulatory T cells (Treg cells) in a diseased tissue;
decreasing the proportion of Th1 and Th17 cells in a diseased tissue; or
inhibiting IFN-γ and IL-17 factors in serum.

In another aspect of the present invention, provided is a hypoxia-treated mesenchymal stem cell or a cell culture or culture supernatant thereof obtained by the following method: continuously culturing mesenchymal stem cells under the condition of 1% to 15%, preferably 1% to 13%, more preferably 5% to 10% of oxygen in terms of volume ratio. Preferably, the culturing is lasted for more than one passage, more preferably for more than three passages, such as from 3 to 20 passages, from 3 to 10 passages, and from 3 to 5 passages.

In another preferred embodiment, the condition of 1% to 15%, preferably 1% to 13%, more preferably 5% to 10% of oxygen in terms of volume ratio refers to: culturing in the air with low oxygen concentration; i.e., in addition to the reduction in the oxygen content and the increase in the nitrogen content in the culture environment thereof, the contents of other proper substances in the air are consistent with those in the air in a conventional normoxic incubator.

In another aspect of the present invention, provided is a pharmaceutical composition for preventing, alleviating or treating inflammatory diseases, comprising an effective amount of the hypoxia-treated mesenchymal stem cell or the cell culture or culture supernatant thereof; and a pharmaceutically acceptable carrier.

In another aspect of the present invention, provided is a method for preparing a hypoxia-treated mesenchymal stem cell or a a cell culture or culture supernatant thereof, comprising: continuously culturing mesenchymal stem cells under the condition of 1% to 15%, preferably 1% to 13%, more preferably 5% to 10% of oxygen in terms of volume ratio.

In another aspect of the present invention, provided is a method for improving the effect of a mesenchymal stem cell or a cell culture or culture supernatant thereof on preventing, alleviating or treating inflammatory diseases, comprising: treating the mesenchymal stem cell with hypoxia.

In another aspect of the present invention, provided is a method for promoting the secretion of insulin-like growth factor-2 by a mesenchymal stem cell, comprising: treating the mesenchymal stem cell with hypoxia. Preferably, the hypoxia is 1% to 15%, preferably 1% to 13%, more preferably 5% to 10% of oxygen by volume.

In another aspect of the present invention, provided is the use of insulin-like growth factor-2 (IGF-2) in the manufacture of a medicament for preventing, alleviating or treating inflammatory diseases. Preferably, the inflammatory diseases include: multiple sclerosis or inflammatory bowel disease (IBD).

In a preferred embodiment, the insulin-like growth factor-2 comprises: an active fragment having or containing positions 25 to 91 of the amino acid sequence of the insulin-like growth factor-2.

In a preferred embodiment, the medicament is also used for:
regulating immune response;
increasing the proportion of regulatory T cells in a diseased tissue;
decreasing the proportion of Th1 and Th17 cells in a diseased tissue; or
reducing the infiltration of inflammatory cells in an inflammatory tissue.

In another aspect of the present invention, provided is a pharmaceutical composition for preventing, alleviating or treating inflammatory diseases, comprising: an effective amount of insulin-like growth factor-2, and a pharmaceutically acceptable carrier. The insulin-like growth factor-2 comprises: an active fragment having or containing positions 25 to 91 of the amino acid sequence of the insulin-like growth factor-2.

In another aspect of the present invention, provided is a kit for medicinal use for preventing, alleviating or treating inflammatory diseases, characterized in that the kit comprises: the hypoxia-treated mesenchymal stem cell or the cell culture or culture supernatant thereof; or the pharmaceutical composition.

Other aspects of the present invention will be apparent to those skilled in the art from the disclosure herein.

### Brief descriptions of the drawings

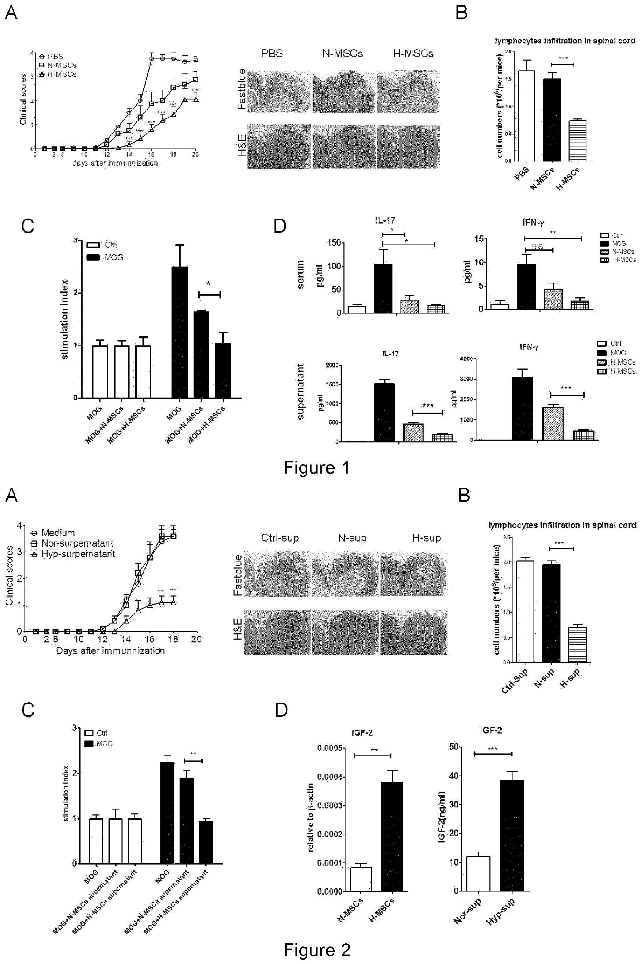

Fig.1 Hypoxia pre-conditioning enhanced the therapeutic effects of mesenchymal stem cells on EAE.
   (A) EAE mice were treated with normoxia-mesenchymal stem cells (N-MSCs) or hypoxia-mesenchymal stem cells (H-MSCs). MSCs (2×10⁵ per mouse) were administered i.v. on days 9, 12, 15 post-induction. Disease severity was scored daily. Mice were euthanized on day 15 post-immunization. Spinal cords from control group (PBS), N-MSCs, H-MSCs were harvested for H&E staining and fast blue-staining to evaluate the immune cells infiltration and demyelination. H-MSCs showed better therapeutic effects on EAE as shown by their abilities in reducing demyelination and mononuclear cell infiltration in the spinal cord. PBS group is the control group that EAE mice were treated with PBS. N-MSCs stand for the group that EAE mice were treated with normoxia-mesenchymal stem cells; H-MSCs stands for the group that EAE mice were treated with hypoxia-mesenchymal stem cells. ***p<0.001.
   (B) Mononuclear cells infiltrating in different treated groups. Mononuclear cells infiltrating into spinal cord from treated or control EAE mice were isolated by percoll gradient and enumerated on day 15 post EAE induction. PBS group is the control group that EAE mice were treated with PBS. N-MSCs stand for the group that EAE mice were treated with normoxia-mesenchymal stem cells; H-MSCs stands for the group that EAE mice were treated with hypoxia-mesenchymal stem cells. ***p<0.001.
   (C) Splenocytes *in vitro* proliferation in different treated groups. Splenocytes derived from control, N-MSCs or H-MSCs treated EAE mice were stimulated with MOG₃₅₋₅₅ (20 µg/ml) for 72 h, and proliferation assessed by [³H]-thymidine incorporation. Stimulation index (SI) was calculated by proliferation of splenocytes activated by MOG₃₅₋₅₅(MOG group)/proliferation of splenocytes without MOG₃₅₋₅₅(Ctrl group). H-MSCs treatment inhibits the MOG₃₅₋₅₅-stimulated T cell proliferation. MOG group is the group that the EAE mice were treated with PBS. N-MSCs stand for the group that EAE mice were treated with normoxia-mesenchymal stem cells; H-MSCs stands for the group that EAE mice were treated with hypoxia-mesenchymal stem cells. *p<0.05.
   (D) The amount of IL-17 and IFN-γ in the EAE mice serum and culture supernatant of splenocytes in different treated groups. The serum of the EAE mice of the control, N-MSCs and H-MSCs groups was collected on day 15 for IL-17 and IFN-γ examination by ELISA. In the mean while, splenocytes from mice were collected and stimulated with MOG₃₅₋₅₅ (20 µg/ml) for 72 h. The levels of IFN-γ and IL-17 in the splenocytes culture supernatant were measured. As shown in the result, EAE mice had high levels of IL-17 and IFN-γ in the serum and MOG₃₅₋₅₅-stimulated splenocytes cultured medium. N-MSCs injection partially suppressed the levels of IFN-γ and IL-17. Importantly, H-MSCs are more effective in reducing these indexes of inflammation. Ctrl group is the group with normal mice; MOG group is the group with EAE mice treated with PBS. N-MSCs stand for the group that EAE mice were treated with normoxia-mesenchymal stem cells; H-MSCs stands for the group that EAE mice were treated with hypoxia-mesenchymal stem cells. N.S., no significance; *p<0.05, **p<0.01, ***p<0.001.
Fig. 2 Culture supernatant from hypoxia pre-conditioned MSCs alleviated EAE.
   (A) Culture supernatant from normoxia-mesenchymal stem cells (N-MSCs) or hypoxia-mesenchymal stem cells was applied to treat EAE. MSCs were cultured under normoxia condition or hypoxia condition and changed to the medium containing 5%FBS at a cell density of 70%. MSC culture supernatant was harvested 48 hours later. Supernatants were concentrated 10-fold and kept the >3 kD proteins. EAE mice were treated by i.p. injection with 200 ul/animal concentrated supernatants (Ctrl-sup, N-sup or H-sup, respectively) from 5% FBS complete medium N-MSCs and H-MSCs,daily from day 9 to 13 post-induction of EAE). Disease severity was scored daily. Spinal cords were harvested on day 15 post-EAE induction, and stained histologically with fast blue or H&E. The graph shows H-sup is effective in treating EAE. Ctrl-sup stands for the group that EAE mice were treated with concentrated 5% FBS complete medium. N-sup stands for the group that EAE mice were treated with N-MSCs culture supernatant. H-sup stands for the group that EAE mice were treated with H-MSCs culture supernatant. **p<0.01.
   (B) Mononuclear cells infiltrating in different treated groups. EAE mice were euthanized on day 15 post EAE induction. Mononuclear cells infiltrating into spinal cords were isolated by percoll gradient and counted. H-sup treatment significant decreases the mononuclear cell infiltration in the spinal cord. Ctrl-sup stands for the group that EAE mice were treated with 5% FBS complete medium. N-sup stands for the group that EAE mice were treated with N-MSCs culture supernatant. H-sup stands for the group that EAE mice were treated with H-MSCs culture supernatant. ***p<0.001
   (C) Splenocytes *in vitro* proliferation in different treated groups. On day 15 post EAE induction, splenocytes derived from Ctrl-sup, H-sup or N-sup treated EAE mice were collected and stimulated with MOG₃₅₋₅₅ (20 µg/ml) for 72 h, and proliferation assessed by [³H]-thymidine incorporation. Stimulation index (SI) was calculated by proliferation of splenocytes activated by MOG₃₅₋₅₅(MOG group)/proliferation of splenocytes without MOG₃₅₋₅₅(Ctrl group). The result showed that H-sup treatment dramatically attenuated the MOG-stimulated T cell proliferation. MOG group is the group that EAE mice were treated with PBS. N-sup stands for the group that EAE mice were treated with N-MSCs culture supernatant. H-sup stands for the group that EAE mice were treated with H-MSCs culture supernatant. **p<0.01.
   (D) mRNA and protein levels of insulin like growth factor-2 (IGF-2) in H-MSCs and N-MSCs. MSCs were cultured under normoxia or hypoxia for three passages to generate N-MSCs and H-MSCs. N-MSCs and H-MSCs were seeded in 6 well plate at the cell density of 50%. N-MSCs and H-MSCs were changed to fresh medium on the next day. IGF-2 mRNA and protein expressions were determined 48h later. H-MSCs have higher IGF-2 expression at both mRNA and protein levels than N-MSCs. N-MSC stands for normoxia conditioned MSCs. H-MSCs stands for hypoxia conditioned MSC. **p<0.01, ***p<0.001.

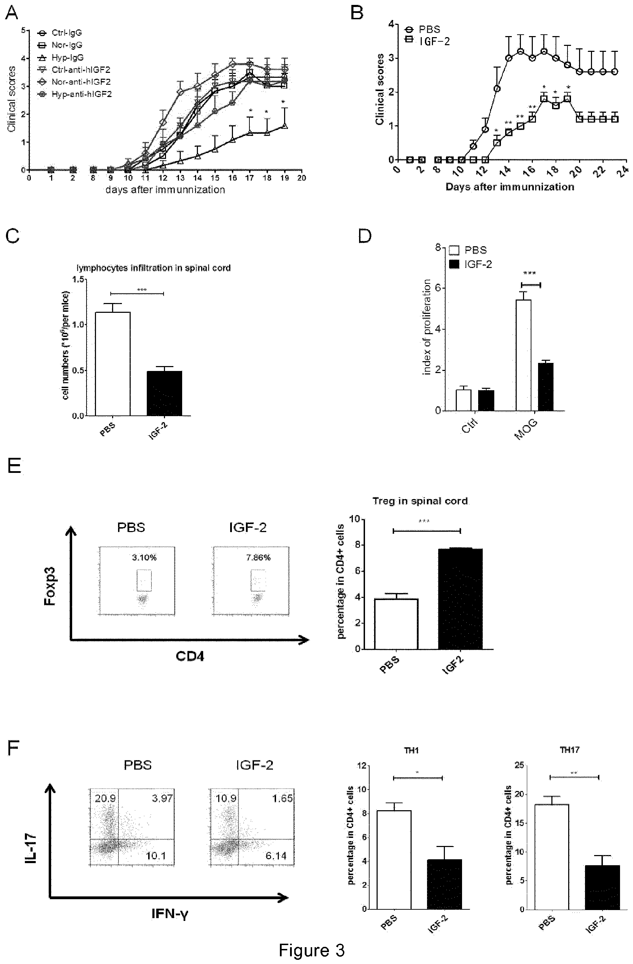

Fig. 3 The effects of IGF-2 and IGF-2 Ala₂₅-Glu₉₁ peptide in treating EAE.
   (A) Neutralize antibody against IGF-2 diminished the therapeutic effects of H-sup. Human IGF-2 (hIGF2) neutralize antibody or their isotype control antibody were co-injected with N-MSCs supernatant or H-MSCs supernatant to treat EAE. The results showed that IGF-2 neutralize antibody suppressed that therapeutic effects of H-MSCs supernatant. Ctrl stands for EAE mice treated with 5% FBS completed medium co-injected. Nor-IgG stands for EAE mice treated with N-MSCs supernatant. Hyp-IgG stands for EAE mice treated with H-MSCs supernatant. Anti-hIGF-2 stands for IGF-2 neutralize antibody. IgG stands for isotype control for IGF-2 neutralize antibody. *p<0.05.
   (B) The effects of IGF-2 Ala₂₅-Glu₉₁ peptide in treating EAE. EAE mice received IGF-2 Ala₂₅-Glu₉₁ peptide (5ng per mouse per day) on day 8 post EAE induction. Clinical scores were evaluated. The result showed that IGF-2 Ala₂₅-Glu₉₁ peptide significantly inhibits the progression of EAE. PBS is control group which EAE mice receive PBS injection. IGF-2 stands for EAE mice treated with IGF-2 Ala₂₅-Glu₉₁ peptide. *p<0.05, **p<0.01.
   (C) Administration of IGF-2 Ala₂₅-Glu₉₁ peptide reduces mononuclear cell infiltration in the focus of EAE. Mononuclear cells infiltrating into spinal cord from treated or control EAE mice were isolated by percoll gradient and enumerated on day 15 post EAE induction. The result showed that IGF-2 Ala₂₅-Glu₉₁ peptide reduces mononuclear cell infiltration in the spinal cord of EAE mice.PBS is control group which EAE mice receive PBS injection. IGF-2 stands for EAE mice treated with IGF-2 Ala₂₅-Glu₉₁ peptide. ***p<0.001.
   (D) IGF-2 Ala₂₅-Glu₉₁ peptide inhibits antigen-specific T cell proliferation. Splenocytes were collected from EAE mice received IGF-2 Ala₂₅-Glu₉₁ peptide treatment on day 15 post EAE induction. Splenocytes were stimulated with MOG₃₅₋₅₅ (20 µg/ml), and proliferation assessed by [³H]-thymidine incorporation. Stimulation index (SI) was calculated by proliferation of splenocytes activated by MOG₃₅₋₅₅(MOG group)/proliferation of splenocytes without MOG₃₅₋₅₅(Ctrl group). The result showed that IGF-2 Ala₂₅-Glu₉₁ treatment dramatically reduces the MOG-stimulated T cell proliferation. PBS is control group which EAE mice receive PBS injection. IGF-2 stands for EAE mice treated with IGF-2 Ala₂₅-Glu₉₁ peptide. ***p<0.001.
   (E) IGF-2 Ala₂₅-Glu₉₁ peptide increases regulatory T cells. Mononuclear cells infiltrating into spinal cord from IGF-2 Ala₂₅-Glu₉₁ peptide treated or control EAE mice were isolated by percoll gradient and on day 15 post EAE induction. CD4⁺Foxp3⁺ T cells were detected by flow cytometry. The results showed that the percentage of Foxp3⁺ cells in spinal cord infiltrated CD4⁺ cells were increased by IGF-2 Ala₂₅-Glu₉₁ peptide treatment. PBS is control group which EAE mice receive PBS injection. IGF-2 stands for EAE mice treated with IGF-2 Ala₂₅-Glu₉₁ peptide. ***p<0.001.
   (F) IGF-2 Ala₂₅-Glu₉₁ peptide decreases Th1 and Th17 cells in the spinal cord of EAE mice. Mononuclear cells infiltrating into spinal cord from IGF-2 Ala₂₅-Glu₉₁ peptide treated or control EAE mice were isolated by percoll gradient and on day 15 post EAE induction. CD4⁺ IL-17⁺ and CD4⁺ IFN-γ⁺ cells were detected by flow cytometry. The results showed that the percentage of IFN-g⁺ and IL-17⁺ cells in spinal cord infiltrated CD4⁺ cells were decreased by IGF-2 Ala₂₅-Glu₉₁ peptide treatment. PBS is control group which EAE mice receive PBS injection. IGF-2 stands for EAE mice treated with IGF-2 Ala₂₅-Glu₉₁ peptide. *p<0.05, **p<0.01.

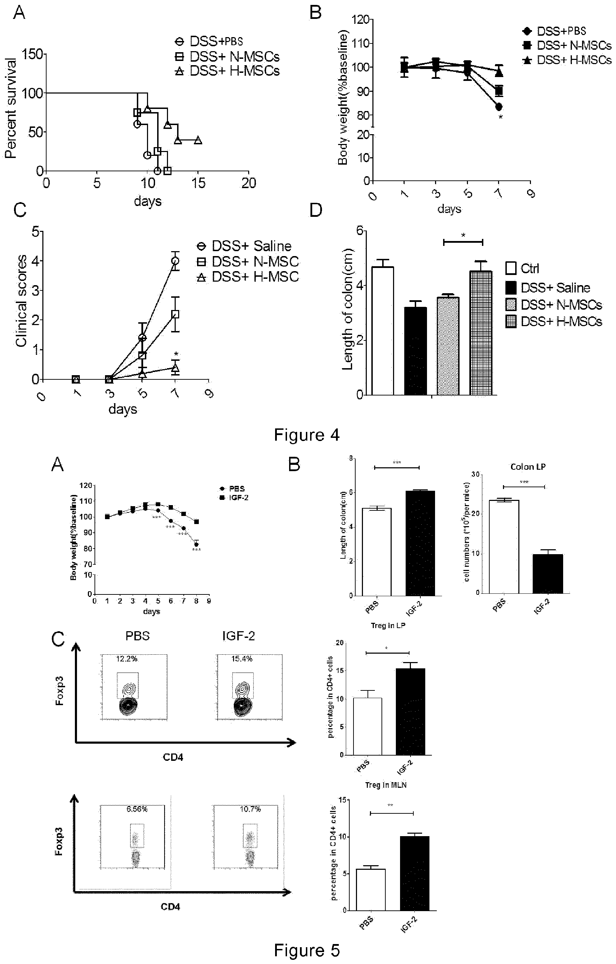

Fig. 4 Hypoxia pre-conditioned MSCSs are effective in treating inflammatory bowel diseases (IBD).
   (A) The survival curve of IBD mice with different treatment. To induce IBD, 3% dextran sodium sulfate (DSS) in water was provided. Normoxia-mesenchymal stem cells (N-MSCs) or hypoxia-mesenchymal stem cells (H-MSCs) (1 × 10⁶/animal each time) were i.p. administrated to treat IBD mice on days 1, 3 and 5. Survival rates were monitored. H-MSCs showed better therapeutic effects than N-MSCs. N-MSCs stands for IBD mice treated with N-MSCs. H-MSCs stands for IBD mice treated with H-MSCs.
   (B) Body weight of IBD mice treated with PBS, N-MSCs or H-MSCs. Normoxia-mesenchymal stem cells (N-MSCs) or hypoxia-mesenchymal stem cells (H-MSCs) (1 × 10⁶/animal each time) were i.p. administrated to treat IBD mice on days 1, 3 and 5. Body weight of IBD mice was measured daily. The average body weight on the first day of IBD induction was defined as baseline. The results showed that H-MSCs treatment better protected IBD mice from body weight loss than N-MSCs and PBS treatment. N-MSCs stands for IBD mice treated with N-MSCs. H-MSCs stands for IBD mice treated with H-MSCs.*p<0.05.
   (C) Clinical score of different treated IBD mice. Normoxia-mesenchymal stem cells (N-MSCs) or hypoxia-mesenchymal stem cells (H-MSCs) (1 × 10⁶/animal each time) were i.p. administrated to treat IBD mice on days 1, 3 and 5. Clinical score of IBD was calculated based on body weight, fecal consistency and bleeding. The average body weight on the first day of IBD induction was defined as baseline. H-MSCs have better therapeutic effects on IBD than N-MSCs. N-MSCs stands for IBD mice treated with N-MSCs. H-MSCs stands for IBD mice treated with H-MSCs. *p<0.05.
   (D) Colon length from H-MSCs or N-MSCs treated EAE mice. Normoxia-mesenchymal stem cells (N-MSCs) or hypoxia-mesenchymal stem cells (H-MSCs) (1 × 10⁶/animal each time) were i.p. administrated to treat IBD mice on days 1, 3 and 5. Mice were euthanized on day 8 post disease inductions to measure colon length. The results showed that H-MSCs treatment is better in alleviating IBD than H-MSCs and PBS treatment. Ctrl group is normal mice. N-MSCs stands for IBD mice treated with N-MSCs. H-MSCs stands for IBD mice treated with H-MSCs. *p<0.05.
Fig. 5 The effect of IGF-2 Ala₂₅-Glu₉₁ peptide is effective in IBD.
   (A) To induce IBD, 3% dextran sodium sulfate (DSS) in water was provided ad libitum for 7 days. IGF-2 Ala₂₅-Glu₉₁ peptide (50 ng per mouse) was i.p. administrated to treat IBD mice daily post IBD induction. Body weight of IBD mice was measured daily. The average body weight on the first day of IBD induction was defined as baseline. IGF-2 Ala₂₅-Glu₉₁ peptide administration protects IBD mice from body weight loss. PBS is control group which EAE mice receive PBS injection. IGF-2 stands for EAE mice treated with IGF-2 Ala₂₅-Glu₉₁ peptide. ***p<0.001.
   (B) Colon length was measured at day 8 post IBD induction. The results showed that IGF-2 Ala₂₅-Glu₉₁ peptide protected IBD mice from colon loss. Mononuclear cells from lamina propria were isolated using percoll gradient and counted. IGF-2 Ala₂₅-Glu₉₁ peptide treatment reduced the mononuclear infiltration in the lamina propria of IBD mice. PBS is control group which EAE mice receive PBS injection. IGF-2 stands for EAE mice treated with IGF-2 Ala₂₅-Glu₉₁ peptide. ***p<0.001.
   (C) Mice were euthanized on day 8 post IBD induction. Mononuclear cells form mesenteric lymph nodes and colon lamina propria was isolated. The percentages of Treg (Foxp3), Th1 (IFN-γ⁺) and Th17 (IL-17⁺) were determined by flowcytometry. IGF-2 Ala₂₅-Glu₉₁ peptide increased Treg cells and reduced the Th1 and Th17 cells in the mesenteric lymph nodes and colon lamina propria. PBS is control group which EAE mice receive PBS injection. IGF-2 stands for EAE mice treated with IGF-2 Ala₂₅-Glu₉₁ peptide. *p<0.05, **p<0.01.

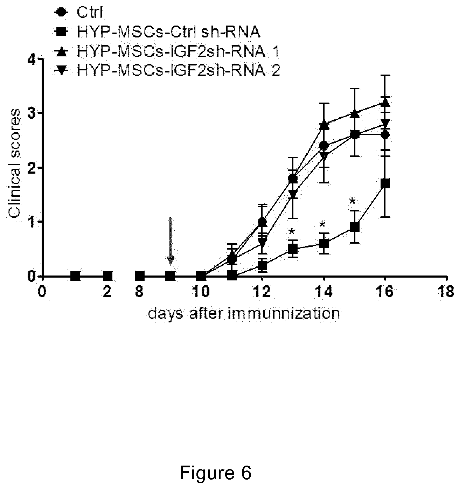

Fig.6 Interfering IGF-2 expression in MSCs affects their therapeutic effects on EAE. MSCs were transfected two different IGF-2 shRNA (shRNA 770 and shRNA 1526) and cultured under normoxia or hypoxia conditions for three passages. These MSCs (1 × 10⁵ per mouse) were i.v. administrated in to EAE mice on day 8 post EAE inductions. The results showed that IGF-2 shRNA diminished the therapeutic effects of H-MSCs. *p<0.05.

### Detailed Description of Embodiments

After an in-depth study, the inventors of the present invention disclose for the first time that treating mesenchymal stem cells with hypoxia can significantly promote alleviating or therapeutic effects of mesenchymal stem cells on inflammatory diseases. The present invention also discloses that the hypoxia-treated mesenchymal stem cell is capable of producing insulin-like growth factor-2, which plays a central role in the treatment of inflammatory diseases with the hypoxia-treated mesenchymal stem cell.

### Hypoxia-treated mesenchymal stem cells and use thereof and a pharmaceutical composition

The inventors of the present invention have selected an animal model of experimental autoimmune encephalomyelitis (EAE), which is an animal model of multiple sclerosis. During cell treatment, mesenchymal stem cells (from human umbilical cord) cultured under normoxia have showed a certain therapeutic effect, and after hypoxic pre-conditioning, the therapeutic effect of mesenchymal stem cells has been further enhanced.

Based on new findings of the inventors of the present invention, the present invention provides MSCs, which are hypoxia-treated MSCs. The preparation method thereof is easy and requires no transgenic manipulations, and the insertion of exogenous genes is not involved. In addition, there is no safety issue during administering.

As a preferred embodiment of the present invention, the hypoxia treatment means a hypoxia environment in which oxygen is 1% to 15%, preferably 1% to 13%, more preferably 5% to 10% by volume. Preferably, in addition to the reduction in the oxygen content and the increase in the nitrogen content in the culture environment thereof, the contents of other proper substances in the air are consistent with those in the air in a conventional normoxic incubator.

Based on the new discovery of the present invention, also provided is the use of hypoxia-treated MSCs in the manufacture of a medicament for preventing, alleviating or treating inflammatory diseases. The inflammatory diseases include, for example, experimental autoimmune encephalomyelitis (EAE) or inflammatory bowel disease (IBD). The hypoxia-treated MSCs are also used for: increasing the proportion of regulatory T cells (Treg cells) in a diseased tissue; decreasing the proportion of Th1 and Th17 cells in a diseased tissue; or inhibiting IFN-γ, IL-17 factors, etc. in serum.

The present invention also provides a composition (medicament) comprising an effective amount (e.g. 0.000001 wt%-50 wt%; preferably 0.00001 wt%-20 wt%; more preferably 0.0001 wt%-10 wt%) of the hypoxia-treated MSCs, and a pharmaceutically acceptable carrier.

As used herein, the term "comprising" means that various components may be applied together to a mixture or composition of the present invention. Thus, the terms "mainly consisting of" and "consisting of' are encompassed by the term "comprising".

As used herein, the term "effective amount" or "effective dose" refers to an amount which can be functional or active to human and/or animals and can be acceptable by human and/or animals.

As used herein, "pharmaceutically acceptable" component refers to a substance which is suitable for use in human and/or mammals without significant adverse side effects (e.g., toxicity, irritation, and allergic reactions), i.e., a substance that has a reasonable benefit/risk ratio. The term "pharmaceutically acceptable carrier" refers to a carrier with which a therapeutic agent is administrated, including various excipients and diluents.

In general, the cells can be formulated into a non-toxic, inert and pharmaceutically acceptable aqueous carrier medium, wherein pH is generally about 5-8, preferably pH is about 6-8.

### Effect of insulin-like growth factor-2 (IGF-2) on the treatment with mesenchymal stem cells

In order to verify whether mesenchymal stem cells exert functions through secreted factors thereof, the inventors of the present invention use the supernatant of mesenchymal stem cells in the treatment of experimental autoimmune encephalomyelitis. The results have showed that only the supernatant of hypoxia-treated cells can exhibit a therapeutic effect, suggesting that hypoxia pre-conditioned mesenchymal stem cells exert a disease treatment function through the secreted factors thereof. For the purpose of further determining which factor(s) plays/play a role in the treatment with hypoxia pre-conditioned mesenchymal stem cells, the inventors of the present invention have examined differences in the expression of mesenchymal stem cell factors cultured under normoxia and hypoxia. The results have showed that the expression quantity of insulin-like growth factor-2 significantly increased after hypoxia treatment.

Insulin-like growth factor-2 is a growth factor mainly secreted by the liver and found in large quantities in the blood. Insulin-like growth factor-2 has anti-apoptotic, growth-regulating, insulin-like and mitogenic functions. It is generally believed that insulin-like growth factor-2 plays an important role in embryonic development and can promote embryonic development and organ formation. It has also been reported that insulin-like growth factor-2 is associated with memory and reproduction. Studies on genetically deficient mice have found that absent signals of insulin-like growth factor-2 can result in impaired brain development. However, there have been no reports about the association of insulin-like growth factor-2 with the treatment of inflammatory diseases so far.

In order to verify whether insulin-like growth factor-2 plays a role in the treatment of experimental autoimmune encephalomyelitis with mesenchymal stem cells, the inventors of the present invention have used neutralizing antibodies to neutralize insulin-like growth factor- 2 in the hypoxia-treated supernatant. After the neutralization, the supernatant shows no efficacy in the disease treatment. After that, the inventors of the present invention have attempted to directly use the Ala25-Glu91 fragment of insulin-like growth factor-2 in the treatment of experimental autoimmune encephalomyelitis. The experimental results show that the use of the Ala25-Glu91 fragment of insulin-like growth factor-2 can effectively treat experimental autoimmune encephalomyelitis. At the site of inflammation, i.e. the spinal cord, the inventors of the present invention have found that the proportion of regulatory T cells (Tregs) has significantly increased, while the proportion of Th1 and Th17 cells has significantly decreased.

It can be seen that for mesenchymal stem cells, hypoxia pre-conditioning can effectively improve the therapeutic effect of experimental autoimmune encephalomyelitis compared to normoxic culturing. The improvement in the therapeutic effect is dependent on the increasing expression of insulin-like growth factor - 2. Using the Ala25-Glu91 fragment of insulin-like growth factor-2 alone can also achieve a good therapeutic effect on experimental autoimmune encephalomyelitis.

Accordingly, the present invention also provides the use of insulin-like growth factor-2 and a Ala25-Glu91 fragment thereof in the manufacture of a composition (medicament) for preventing, alleviating or treating inflammatory diseases.

The insulin-like growth factor-2 comprises full-length insulin-like growth factor-2 or a biologically active fragment thereof. Preferably, the full-length amino acid sequence of the insulin-like growth factor-2 may be substantially identical to the sequence shown in SEQ ID NO: 8 (NCBI protein database no. P01344.1).

The amino acid sequence of insulin-like growth factor-2 formed by the substitution, deletion or addition of one or more amino acid residues is also included in the present invention. The insulin-like growth factor-2 or a biologically active fragment thereof comprises a portion of an alternative sequence of conserved amino acids, and the sequence after amino acid substitution does not affect the activity or retains some of the activity thereof. Proper amino acid substitutions are well-known techniques in the art, which can be easily implemented and ensure that the biological activity of the resulting molecule remains unchanged. These techniques enable a person skilled in the art to recognize that in general, changing a single amino acid in an unnecessary region of a polypeptide does not substantially change its biological activity. See Watson et al., Molecular Biology of The Gene, 4th Edition, 1987, The Benjamin/Cummings Pub. Co. P 224.

Any insulin-like growth factor-2 and biologically active fragment containing positions 25 to 91 of the amino acid sequence thereof can be applied to the present invention. Herein, the biologically active fragment of insulin-like growth factor-2 means the biologically active fragment, as a polypeptide, still retains all or some of the functions of the full-length insulin-like growth factor-2. The biologically active fragment retains at least 50% of the activity of full-length insulin-like growth factor-2 under normal conditions. Under more preferred conditions, the active fragment is capable of retaining 60%, 70%, 80%, 90%, 95%, 99%, or 100% of the activity of full-length insulin-like growth factor-2.

The present invention may also employ modified or improved insulin-like growth factor-2 or an active fragment based on positions 25 to 91 of the amino acid sequence thereof. For example, in order to enhance half-life, effectiveness, metabolism, and/or protein efficacy, it is modified or improved against inflammatory diseases.

The present invention also provides a composition (medicament) comprising an effective amount (e.g. 0.000001 wt%-50 wt%; preferably 0.00001 wt%-20 wt%; more preferably 0.0001 wt%-10 wt%) of the insulin-like growth factor-2, and a pharmaceutically acceptable carrier.

In view of lacking knowledge about the performance of hypoxia-treated mesenchymal stem cells in treating immune-associated diseases in the art, the present invention discloses for the first time that the therapeutic effect of hypoxia-treated mesenchymal stem cells on inflammatory diseases is better than that of mesenchymal stem cells cultured under normoxia. It has also been found that using insulin-like growth factor-2 alone can also achieve a good therapeutic effect on experimental autoimmune encephalomyelitis. The inventors of the present invention have believed that the above findings provide new ideas for elucidating the mechanism of mesenchymal stem cells in the treatment of immune-associated diseases such as autoimmune polio, and also provide new information for further recognizing the value of insulin-like growth factor-2 in the treatment of immune-associated diseases.

The invention is further described in conjunction with particular examples. It should be understood that these examples are merely used for describing the present invention, rather than limiting the scope of the present invention. The experimental methods not specified for the specific conditions in the following examples are generally carried out in accordance with conventional conditions, such as the conditions described in J. Sambrook et al. (eds), Molecular Cloning: A Laboratory Manual, 3rd Edition, Science Press, 2002, or in accordance with the conditions recommended by the manufacturer.

### Material and Method

All agents and material are commercially available unless otherwise stated. wherein the source of the materials are as follows:
Recombinant human IGF-2 Ala₂₅-Glu₉₁, IGF-2 neutralizing antibodies and the control IgG were purchased from R&D system. Myelin oligodendrocyte glycoprotein (MOG₃₅₋₅₅) was purchased from GL Biochem (Shanghai, China). Incomplete freund adjuvant (IFA) and M. Tuberculosis (TB) were purchased from Sigma-Aldrich (MO, USA). Pertussis toxin was purchased from List Biological Laboratories(USA). Dextran sodium sulfate (DSS) was purchased from MP Biomedicals. Human IGF-2 ELISA kit was purchased from Mediagnost (German). Mouse IFN-γ□ and IL-17 ELISA kits were purchased from R&D systems. Anti-mouse CD4 PerCP-Cy5.5, anti-mouse CD4 PerCP-Cy5.5, anti-mouse IL-17A PE and anti-mouse IL-17A PE for flow cytometry were purchased from Ebioscience.

C57BL/6 mice were purchased from Shanghai SLAC Laboratory Animal Co.,Ltd and maintained in the experimental animal science department of the Shanghai Jiao Tong University School of Medicine.

### 1. Cell preparation.

Isolation and culture of human umbilical cord derived MSCs: Blood vessels were removed from the umbilical cord. The umbilical cord tissue was dissected into pieces and put into culture dish to allow the MSCs released from the tissue. The medium was changed every two days until the cells reached to a certain density. Cells were digested and passaged for further experiment.

Preparation of hypoxia conditioned MSCs: MSCs were incubated in a thermo low oxygen incubator. The agents used were identical to the culture under the normoxia conditioned culture except for the change of incubator. Hypoxia condition: 10% O₂ (v/v).

Construction of IGF-2 knockdown MSCs: IGF-2 expression was knocked down using lentivirus transfection. The control shRNA target sequence: Ctrl-sh-RNA ( 5'-ttctccgaacgtgtcacgt-3' (SEQ ID NO: 1)); the shRNA target sequences for IGF-2: sh-RNA-770 (5'- gaagtcgatgctggtgcttct -3' (SEQ ID NO: 2)); sh-RNA-1526 (5'-gctttaaacacccttcacata -3' (SEQ ID NO: 3)). The lentivirus virus vector carrying GFP and puromycin resistance gene. The infected cells showed green fluorescence. In the knowdown experiment, the virus was added to the medium when MSCs reached a density of 60%. MSCs were changed to normal culture medium 24 hours after virus infection. The efficiency of infection could be speculated through counting GFP positive cells under a fluorescence microscope. Puromycin was added to the culture medium 24 hours later. Cells were passaged for further experiment.

### 2. EAE induction and experimental therapies

### (1). Preparations before the experiment

Prepare complete freund adjuvant(CFA): Supply heat-inactivated M. Tuberculosis (5 mg/ml) into incomplete freund adjuvant. Blend the mixture well through upside down before use.

Antigen emulsification: Carefully connect the two syringes via the three-pass-connecter. Place MOG (300 ug in 100 ul PBS) and 100 ul complete freund adjuvant (CFA) into a syringe. Drive out the air bubbles. Prepare MOG-CFA solution by pushing the syringes for 500 times with increasing resistance, thereby various components are emulsified.

Prepare Pertussis toxin: Dilute pertussis toxin to a working concentration of 1 ng/ul.

### (2). EAE induction and experimental therapies

Day 0, 200 ul emulsificated antigens was inoculated subcutaneously on the back of mouse, 100 ul at each side of the back corresponding to the chest. 200 ul/animal pertussis toxin was tail intravenous administration into mouse.

Day 2, 200 ul/animal pertussis toxin was intravenous administration into mouse.

Day 8, 11, and 14, MSCs (2.5 × 10⁵) were i.v. administrated to the tail of EAE mice; Control group received 200 ul Saline.

MSCs supernatant was injected from day 9 to 13 when MSCs supernatant was used for therapy.

EAE mice were intraperitoneal injected with 5 ng/animal IGF-2 Ala₂₅-Glu₉₁ daily from day 8.

Preparation of hypoxia conditioned MSCs: MSCs were cultured under 10%(v/v) oxygen for three passages. 10% oxygen was established in Forma™ Series II 3110 Water-Jacketed CO2 Incubator through controlling nitrogen concentration.

### (3). Clinical Scoring of EAE

0: Clinically normal;
0.5: Partial tail paralysis;
1: Tail paralysis;
2. Hind limb weakness;
3. Paralysis in one hind limb;
4. Paralysis in both hind limbs;
5. Moribund.
3. IBD Induction and experimental therapies

DSS solution was prepared at a proportion of 2.5:100 (w/v)and was sterilized through 22 um filterer to ensure bacteria free, sealed the DSS in the tube before use.

8∼10w female C57BL/6 mice were selected. The prepared DSS solution was provided to the 8∼10w female C57BL/6 instead of the drinking water to induce IBD. Exam the mice body weight and fecal daily, supplied new DSS every other day.

MSCs treated under normoxia condition or hypoxia condition (2 × 10⁶cells/animal) were i.p. administrated to mice on days 1, 3 and 5.

50 ng/animal IGF-2 Ala₂₅-Glu₉₁ was injected i.p. daily into mice.

### 4. Splenocytes in vitro proliferation assay

Euthanized EAE mice and make spleen into single cell suspension. Splenocytes from EAE mice were seeded in 96 U bottom plates (5×10⁵cells/well) and stimulated with the 20 ug/ul MOG₃₅₋₅₅.

72 hours after incubated in the 37□ incubator, ³H labeled thymine was added. 6 hours later, repeated freezing and thawing and the cells were attached onto the Special filter membrane via vac-sorb. Cpm value was obtained from Wallac MicroBeta liquid scintillation counting instrument and the amount of ³H thymine incorporation was determined.

### 5. Determine the gene express by Real time PCR

Total RNA was extracted using a TIANGEN RNA cell RNA extract Kit and reverse-transcribed to cDNA according to the instruction of TIANGEN TIANScript first-strand cDNA kit.. The takara fluorescent quantitative PCR agent was added into PCR reaction according to the instruction. The gene expression levels were normalized to the expression of β-actin through calculating 2^{-ΔΔCT}. The expression of the genes were converted to the folds of β-actin expression. Primer sequences were as follows:
Human IGF-2:
   Forward: 5'-CTTGGACTTTGAGTCAAATTGG-3' (SEQ ID NO: 4);
   Reverse: 5'-GGTCGTGCCAATTACATTTCA-3' (SEQ ID NO: 5);
Human□ β-actin:
   Forward: 5'-TTGCCGACAGGATGCAGAAGGA-3' (SEQ ID NO: 6);
   Reverse: 5'-AGGTGGACAGCGAGGCCAGGAT-3' (SEQ ID NO: 7).

### 6. Intracellular immunofluorescence staining for cytokine or Foxp3

(1) All cells (about 5∼10×10⁵) were washed with PBS for one time. Blocked by incubation with anti-mouse CD 16/32 antibody on ice for 10 minutes, 30 ul/tube.
(2) Surface marker staining: Suspend 1×10⁶ cells in 100 ul FACS buffer and add fluorophore-conjugated antibody, incubate in the dark for 30min at 4□.
(3) Wash the cells with FACS buffer and add 100 ul/tube Fixation/Permeabilization buffer. Place at 4 °Covernight to fix the cells.
(4) Centrifuge at 400×g for 5 min and suspended the cells with 200ul 1x Permeabilization buffer, mix well.
(5) Centrifuge at 400×g for 5 min and resupend the cells with 50 ul/tube 1x Permeabilization containing anti-cytokine antibody or anti-Foxp3 antibody. Stain on ice for 1 hour in the dark.
(6) Add 200ul 1×Permeabilization buffer and mix well. Centrifuge at 400×g for 5 minutes.
(7) Discard the supernatant and add 200ul 1×Permeabilization buffer, mix well. Centrifuge at 400×g for 5 minutes.
(8) Discard the supernatant and add 200 ul FACS buffer, mix well. Centrifuge at 400×g for 5 minutes. Re resupend the cell pellet with 200ul FACS buffer before flow cytometry analysis.

### 7. Data processing and statistical analysis

Each group of the experiment results had three or more than three samples. Graphpad Prism 5 was used for charting. The data in the figure was showed by standard error of the mean (SEM). Student's test was used for data processing. Significant difference in means is indicated thusly: α=0.05, *p < 0.05, **p < 0.01 and ***p < 0.001.

### Example 1 Hypoxia pre-conditioning enhanced the therapeutic effects of mesenchymal stem cells on experimental autoimmune encephalomyelitis (EAE)

To evaluate the influence of hypoxia pre-conditioning on the therapeutic effects of MSCs on EAE, MSCs were cultured under 10% oxygen for more than three passages and then used for therapy on days 9, 12, 15 post EAE induction.

The inventor found that hypoxia pre-conditioned MSCs were significantly more effective in the therapy of EAE. This was also evidenced by dramatic decreases in demyelination and mononuclear cell infiltration in the spinal cord compared to normoxia conditioned MSCs treatment (Figure 1A and 1B).

*In Vitro* Experiment of splenocytes proliferation showed that splenocytes harvested from hypoxia pre-conditioned MSCs treated EAE mice significantly reduced T-cell proliferation to MOG stimulation in vitro (Figure 1C).

Detection of the serum of mice showed that the levels of the inflammatory cytokines such as IFN-γ□ and IL-17 in serum of EAE mice were inhibited significantly after treated with the hypoxia pre-conditioned MSCs (Figure 1D).
Taken together, MSCs did exhibit a certain effect on EAE. Hypoxia pre-conditioning can dramatically enhance the therapeutic effects of MSCs and alleviate disease symptoms.

### Example 2 Culture supernatant of hypoxia pre-conditioned MSCs effectively treat EAE

These studies were conducted to determine the effects of hypoxia pre-conditioned mesenchymal stem cells (MSCs) secretome on EAE. The inventor employed supernatant from normoxia-mesenchymal stem cells or hypoxia-mesenchymal stem cells to treat EAE. (The culture supernatant of MSCs was i.p. administrated to mouse every day from day 9 to 13 post EAE inductions.)

The results showed that supernatant from normoxia-mesenchymal stem cells do not have therapeutic effects on EAE. While supernatant from hypoxia-mesenchymal stem cells have dramatic therapeutic effects on EAE (Figure 2A).

As with the effects of hypoxia pre-conditioned MSCs, supernatant from hypoxia-mesenchymal stem cells dramatically inhibited demyelination and immune cells infiltration (Figure 2A and 2B). MOG-stimulated proliferations were also suppressed by supernatant from hypoxia-mesenchymal stem cells (Figure 2C).

Taken together, hypoxia pre-conditioned MSCs exert their therapeutic effects on EAE through secreted factors.

Therefore, the inventor profiled the gene expression in normoxia and hypoxia MSCs by microarray analysis. The inventor found that insulin like growth factor-2 (IGF-2) was highly expressed in hypoxia pre-conditioned MSCs but not normoxia conditioned MSCs. The high levels of IGF-2 expression in hypoxia pre-conditioned MSCs were validated by real time PCR and ELISA (Figure 2D).

### Example 3 Insulin like growth factor-2 plays a key role in the therapeutic effects of hypoxia pre-conditioned MSCs on EAE

To investigate the roles of insulin like growth factor-2 (IGF-2) in MSCs therapy, IGF-2 neutralize antibody (R&D) was applied to block the effects of IGF-2 in MSCs supernatant. The injection of IGF-2 neutralize antibody (5 ng /mouse / day) diminished the therapeutic effects of supernatant from hypoxia-mesenchymal stem cells (Figure 3A). Besides, the inventor also employed shRNA 770 and shRNA 1526 (purchased from Genepharma) to interfere IGF-2 expression in MSCs. As showed in Figure 6, compared to MSCs treated with control shRNA, MSCs transfected with IGF-2 shRNA were also no longer effective in alleviating EAE (Figure 6). Therefore, IGF-2 is the key factor mediating the therapeutic effects of hypoxia pre-conditioned MSCs on EAE.

To further define the role of IGF-2, the inventor employed IGF-2 Ala₂₅-Glu₉₁ peptide from R&D to treat EAE from day 8 post EAE induction (5 ng per animal) and found that IGF-2 Ala₂₅-Glu₉₁ peptide significantly inhibit EAE as shown by lowered the EAE clinical scores (Figure 3B), lessened mononuclear cell infiltration in the CNS (Figure 3C) and reduced MOG-stimulated T cell proliferation (Figure 3D).

Notably, IGF-2 Ala₂₅-Glu₉₁ peptide treatment increases the CD4⁺Foxp3⁺ regulatory T cells in the spinal cord of mouse (Figure 3E) and decrease Th1 and Th17 cells dramatically (Figure 3F). These results suggest that IGF-2 Ala₂₅-Glu₉₁ peptide are capable of regulating immune response and alleviate EAE.

### Example 4 Hypoxia pre-conditioned MSCs and IGF-2 Ala₂₅-Glu₉₁, peptide are both effective in alleviating inflammatory bowel diseases (IBD)

Similar to effects observed in EAE model, hypoxia pre-conditioned mesenchymal stem cells (MSCs) showed dramatic therapeutic effects on inflammatory bowel disease (IBD). Hypoxia pre-conditioned MSCs prolonged the survival (Figure 4A), attenuated the body weight loss (Figure 4B) and improved the clinical score (Figure 4C) of IBD mice. Hypoxia pre-conditioned MSCs also protect colon from damage which evidenced by calculating the colon length (Figure 4D).

Our further studies showed that insulin like growth factor-2 (IGF-2) Ala₂₅-Glu₉₁ peptide is effective in alleviating IBD. Sole use of IGF-2 Ala₂₅-Glu₉₁ peptide treatment can attenuated the body weight loss (Figure 5A), prevented the colon shortening and reduced the mononuclear cells infiltration in IBD mice (Figure 5B). The inventor also analyzed the lymphocytes in the inflamed sites including mesenteric lymph node and lamina propia. As with observation in EAE mice, IGF-2 Ala₂₅-Glu₉₁ peptide treatment upregulated CD4⁺ Foxp3⁺ Treg cells and reduced Th1 and Th17 cell (Figure 5C). Therefore, IGF-2 Ala₂₅-Glu₉₁ peptide is capable of modulating immune response to treat certain diseases.

It should be noted that, in the present invention, all of the documents referred to in this application by reference, as if each reference were individually incorporated by reference that. It should also be understood that the above specific embodiments of the present invention and by the use of technical principles, after reading the contents of the present invention described above, the person skilled in the art can make various modifications of the present invention or modifications without departing from the invention. The spirit and scope of these equivalent forms also fall within the scope of the present invention.

## Claims

1. Use of a hypoxia-treated mesenchymal stem cell or a cell culture or culture supernatant thereof in the manufacture of a medicament for preventing, alleviating or treating inflammatory diseases.

2. The use of claim 1, **characterized in that** the inflammatory diseases include: multiple sclerosis or inflammatory bowel disease.

3. The use of claim 1, **characterized in that** the hypoxia is 1%-15% of oxygen by volume.

4. The use of claim 1, **characterized in that** the medicament is also used for:
increasing the proportion of regulatory T cells in a diseased tissue;
decreasing the proportion of Th1 and Th17 cells in a diseased tissue; or
inhibiting IFN-γ and IL-17 factors in serum.

5. A hypoxia-treated mesenchymal stem cell or a cell culture or culture supernatant thereof, **characterized in that** the hypoxia-treated mesenchymal stem cell or the cell culture or culture supernatant thereof is obtained by the following method: continuously culturing mesenchymal stem cells under the condition of 1% to 15% of oxygen in terms of volume ratio.

6. The hypoxia-treated mesenchymal stem cell or the cell culture or culture supernatant thereof in claim 5, **characterized in that** the culturing is lasted for more than one passage, more preferably for more than three passages.

7. A pharmaceutical composition for preventing, alleviating or treating inflammatory diseases, **characterized by** comprising an effective amount of the hypoxia-treated mesenchymal stem cell or the cell culture or culture supernatant thereof in claim 5 or 6, and a pharmaceutically acceptable carrier.

8. A method for preparing a hypoxia-treated mesenchymal stem cell or a cell culture or culture supernatant thereof, **characterized in that** the method comprises: continuously culturing mesenchymal stem cells under the condition of 1% to 15% of oxygen in terms of volume ratio.

9. A method for improving the effect of a mesenchymal stem cell or a cell culture or culture supernatant thereof on preventing, alleviating or treating inflammatory diseases, **characterized in that** the method comprises: treating the mesenchymal stem cell with hypoxia.

10. A method for promoting the secretion of insulin-like growth factor-2 by a mesenchymal stem cell, **characterized in that** the method comprises: treating the mesenchymal stem cell with hypoxia.

11. The method of claim 9 or 10, **characterized in that** the hypoxia is 1%-15% of oxygen by volume.

12. Use of insulin-like growth factor-2 in the manufacture of a medicament for preventing, alleviating or treating inflammatory diseases.

13. The use of claim 12, **characterized in that** the insulin-like growth factor-2 comprises: an active fragment containing positions 25 to 91 of the amino acid sequence of the insulin-like growth factor-2.

14. The use of claim 12, **characterized in that** the inflammatory diseases include: multiple sclerosis or inflammatory bowel disease.

15. The use of claim 12, **characterized in that** the medicament is also used for:
regulating immune response;
increasing the proportion of regulatory T cells in a diseased tissue;
decreasing the proportion of Th1 and Th17 cells in a diseased tissue; or
reducing the infiltration of inflammatory cells in an inflammatory tissue.

16. A pharmaceutical composition for preventing, alleviating or treating inflammatory diseases, **characterized in that** the pharmaceutical composition comprises: an effective amount of insulin-like growth factor-2, and a pharmaceutically acceptable carrier.

17. A kit for preventing, alleviating or treating inflammatory diseases, **characterized in that** the kit comprises: the hypoxia-treated mesenchymal stem cell or the cell culture or culture supernatant thereof in claim 5 or 6; or the pharmaceutical composition of claim 7; or the pharmaceutical composition of claim 16.


Sequence listing

<110>  SHANGHAI INSTITUTES FOR BIOLOGICAL SCIENCES, CHINESE ACADEMY OF
SCIENCES

<120>  LOW-OXYGEN-TREATED MESENCHYMAL STEM CELL AND USE THEREOF

<130>  152537 PCWO

<160>  8

<170>  PatentIn version 3.3

<210>  1
<211>  19
<212>  DNA
<213>  Artificial sequence


<220>
<221>  misc_feature
<223>  Polynucleotide

<400>  1
ttctccgaac gtgtcacgt                                                    19


<210>  2
<211>  21
<212>  DNA
<213>  Artificial sequence


<220>
<221>  misc_feature
<223>  Polynucleotide

<400>  2
gaagtcgatg ctggtgcttc t                                                 21


<210>  3
<211>  21
<212>  DNA
<213>  Artificial sequence


<220>
<221>  misc_feature
<223>  Polynucleotide

<400>  3
gctttaaaca cccttcacat a                                                 21


<210>  4
<211>  22
<212>  DNA
<213>  Artificial sequence


<220>
<221>  misc_feature

<223>  Polynucleotide

<400>  4
cttggacttt gagtcaaatt gg                                                22

<210>  5
<211>  21
<212>  DNA
<213>  Artificial sequence

<220>
<221>  misc_feature
<223>  Polynucleotide

<400>  5
ggtcgtgcca attacatttc a                                                 21

<210>  6
<211>  22
<212>  DNA
<213>  Artificial sequence

<220>
<221>  misc_feature
<223>  Polynucleotide

<400>  6
ttgccgacag gatgcagaag ga                                                22

<210>  7
<211>  22
<212>  DNA
<213>  Artificial sequence

<220>
<221>  misc_feature
<223>  Polynucleotide

<400>  7
aggtggacag cgaggccagg at                                                22

<210>  8
<211>  180
<212>  PRT
<213>  Homo Sapiens

<400>  8